# EUROPEAN PATENT APPLICATION

(11) **EP 4 786 488 A1**
(43) Date of publication of application: **05.08.2026**
(21) Application number: 24870925.5
(22) Date of filing: 27.09.2024
(51) Int. Cl.: C07K 14/47, C07K 14/435, C07K 19/00, A61L 27/52, A61L 27/20, A61L 27/24, C08L 5/08, A61L 27/22, C08J 3/24

(54) **MODIFIED FERRITIN, CROSS-LINKED FERRITIN, AND PREPARATION METHOD AND USE**

(30) Priority: 28.09.2023 CN 202311275385; 14.09.2024 CN 202411296283
(71) Applicant: Shanghai Shangzeyonghi Biotechnology Partnership (Limited Partnership), Shanghai 201306 (CN)
(72) Inventor: SU, Yong, Shanghai 201306 (CN)
(74) Representative: Greaves Brewster LLP
(86) International application number: PCT/CN2024/121622
(87) International publication number: WO 2025/067387

(57) **Abstract**

A modified ferritin, a cross-linked ferritin, and a preparation method and a use. The modified ferritin comprises a plurality of light chain subunits or heavy chain subunits of a naturally-derived ferritin, wherein the plurality of light chain subunits or the plurality of heavy chain subunits form a polymer, preferably a cage-like polymer. The cross-linked ferritin is a naturally-derived ferritin or a variant thereof, or a cross-linked ferritin formed by cross-linking the modified ferritin. The cross-linked ferritin still maintains the unique cage-like structure of the ferritin, and has characteristics such as good elasticity, high degree of support, low degree of swelling, and degradation resistance, achieving better dermal and tissue filling effects.

## Description

The present application claims priority to Chinese Patent Application No. 2023112753858, filed on September 28, 2023, and Chinese Patent Application No. 2024112962839, filed on September 14, 2024. The present application incorporates the full texts of the aforementioned Chinese patent applications by reference.

### TECHNICAL FIELD

The present disclosure relates to the technical field of biomedical materials, and specifically relates to modified ferritin, cross-linked ferritin, a preparation method therefor, and a use thereof.

### BACKGROUND

The skin is the largest organ of the human body and plays an important role in resisting external aggression and maintaining the stability of the internal environment. As age increases, skin aging leads to phenomena such as facial tissue laxity and the appearance of wrinkles. Through subcutaneous filling, it is possible to effectively alleviate facial sagging and drooping, supplement volume loss, reshape facial contours, and improve skin quality. Local injection of hyaluronic acid and/or collagen fillers has become a commonly used method due to advantages such as ease of operation, minimal trauma, and good biocompatibility.

Hyaluronic acid, also known as sodium hyaluronate, is one of the most frequently used dermal fillers in clinical practice, offering advantages such as good tissue compatibility, ease of injection, immediate results post-injection, and being minimally invasive. Because hyaluronic acid is an acidic mucopolysaccharide, it cannot form a three-dimensional spatial structure after cross-linking; consequently, its supportive capacity after filling is limited, and it is prone to the Tyndall effect, which affects the post-filling appearance. Hyaluronic acid is a high-molecular polymer with hydroxyl and carboxyl groups in its chemical structure, making it prone to hydrolysis in the body after interacting with water. Biological enzymes (especially hyaluronidase and human free radicals) have a strong degrading effect on hyaluronic acid. Therefore, the retention time of hyaluronic acid in the body is relatively short, requiring repeated injections to achieve therapeutic efficacy. Meanwhile, skin cells absorb hyaluronic acid very quickly upon initial injection, and staying up late or consuming alcohol can accelerate the metabolism of hyaluronic acid within the body. Furthermore, the metabolic rate of hyaluronic acid accelerates in frequently moved areas. When used as a subcutaneous filler, hyaluronic acid generally has a maintenance period of 6 to 8 months. Additionally, physical impacts can easily cause the displacement of hyaluronic acid or changes in its shape. Therefore, as a subcutaneous filling material, hyaluronic acid still possesses certain defects that require further product improvement.

Collagen is another common filling substance, featuring the advantages of good histocompatibility, ease of injection, immediate results post-injection, and being minimally invasive. The natural collagen existing in human skin is Type I collagen. The amino acid sequence of this collagen contains three uncommon amino acids: 4-hydroxyproline (9%), 3-hydroxyproline (0.1%), and 5-hydroxylysine (0.6%). Through amino acid modification, stable covalent bond linkages can be formed, further forming a right-handed triple-helical protocollagen molecule. Since each individual strand of the protocollagen molecule is a left-handed helix, the natural collagen existing in the skin possesses powerful supportive force and tensile strength. The subcutaneous filling collagens currently available on the market are mostly collagen fragments expressed in prokaryotes, which can form neither the left-handed helical single strands nor the right-handed triple helix. Consequently, they lack the powerful supportive force and tensile strength of collagen in the body and cannot perform their true functions. Furthermore, because the filler consists of collagen fragments, it is easily hydrolyzed by collagenases in the body, resulting in a short retention time *in vivo*.

In view of the shortcomings of hyaluronic acid and collagen, such as limited supportive force and susceptibility to degradation, there is an urgent need to develop novel filling materials that are long-lasting, possess better biomechanical support, and can be produced through large-scale industrialization.

Ferritin is a hollow spherical protein naturally existing in the animal body, with an inner diameter of 8 nm and an outer diameter of 12 nm. It consists of a 24-mer protein cage structure formed by the self-assembly of light chain subunits and heavy chain subunits. In current research, ferritin is often utilized in tissue engineering or as a delivery vehicle for small molecule drugs, biological agents, nucleic acids, and the like; there are few reports regarding its relevant research in the preparation of filling materials.

### SUMMARY

To address the aforementioned defects existing in the prior art, the present disclosure provides a modified ferritin, a cross-linked ferritin, and a preparation method therefor and a use thereof; the prepared cross-linked ferritin possesses good biocompatibility and, in practical application, exhibits excellent properties such as support, degradation resistance, and elasticity.

To achieve the above objectives, the technical solutions adopted by the present disclosure are as follows:

The present disclosure provides a modified ferritin comprising a plurality of light chain subunits or heavy chain subunits of ferritin of natural origin, wherein the plurality of light chain subunits or the plurality of heavy chain subunits form a multimer, preferably a cage-shaped multimer.

In the present disclosure, the term "modified ferritin" refers to non-naturally occurring ferritin, including, for example, artificial ferritin obtained after genetic engineering of naturally derived ferritin. In the present disclosure, the amino acid sequence of the light chain subunit may be as set forth in SEQ ID NO: 1 or have at least 80% identity, preferably at least 85%, 90%, 95%, 96%, 97%, 98%, or 99% identity, with the sequence as set forth in SEQ ID NO: 1.

In the present disclosure, the amino acid sequence of the heavy chain subunit may be as set forth in SEQ ID NO: 2, 6, or 7 or have at least 80% identity, preferably at least 85%, 90%, 95%, 96%, 97%, 98%, or 99% identity, with the sequence as set forth in SEQ ID NO: 2, 6, or 7.

In some embodiments, the modified ferritin comprises 8, 16, 24, or 48 light chain subunits or 8, 16, 24, or 48 heavy chain subunits.

In some embodiments, the natural origin comprises animal, plant, fungal, or bacterial origins, preferably animal origins, the animal being a vertebrate, preferably a mammal, and more preferably a human.

In some preferred embodiments, the modified ferritin consists of 24 light chain subunits forming a cage-shaped multimer, or consists of 24 heavy chain subunits forming a cage-shaped multimer.

The present disclosure further provides a cross-linked ferritin, which is a cross-linked ferritin formed by cross-linking ferritin of natural origin or a variant thereof, wherein the cross-linked ferritin retains the multimeric structure of the ferritin of natural origin or the variant thereof; or is a cross-linked ferritin formed by cross-linking the modified ferritin as described above, wherein the cross-linked ferritin retains the multimeric structure of the modified ferritin; wherein the variant has at least 80% identity, preferably at least 85%, 90%, 95%, 96%, 97%, 98%, or 99% identity, with the amino acid sequence of the ferritin of natural origin.

Preferably, the cross-linked ferritin is in the form of a hydrogel or microsphere structure.

In the present disclosure, the degree of swelling of the cross-linked ferritin may be lower than 1000%, preferably lower than 600%, and more preferably lower than 500%.

In the present disclosure, the elastic modulus of the cross-linked ferritin may be higher than 100 Pa, preferably higher than 600 Pa, and more preferably higher than 1000 Pa.

In the present disclosure, the viscous modulus of the cross-linked ferritin may be higher than 10 Pa, preferably higher than 30 Pa, more preferably higher than 50 Pa, and even more preferably higher than 100 Pa.

In the present disclosure, the ferritin of natural origin may comprise ferritins of animal, plant, fungal, or bacterial origins, preferably of animal origins, the animal being a vertebrate, preferably a mammal, and more preferably a human.

Preferably, the cross-linking is performed via a biological cross-linking reaction or a chemical cross-linking reaction.

In some embodiments, the ferritin is mouse heavy chain ferritin, preferably having an amino acid sequence with NCBI accession number NP_034369.1.

In some embodiments, the ferritin is mouse light chain ferritin, preferably having an amino acid sequence with NCBI accession number AAA37614.1.

In some embodiments, the ferritin is *Drosophila melanogaster* heavy chain ferritin, preferably having an amino acid sequence with NCBI accession number NP_001247366.1.

In some embodiments, the ferritin is *Drosophila melanogaster* light chain ferritin, preferably having an amino acid sequence with NCBI accession number NP_001263105.1.

In some embodiments, the ferritin is *Caenorhabditis elegans* ferritin, preferably having an amino acid sequence with NCBI accession number NP_491198.1.

In some embodiments, the ferritin is oyster ferritin, preferably having an amino acid sequence as set forth in SEQ ID NO: 3.

In some embodiments, the ferritin is human mitochondrial ferritin, preferably having an amino acid sequence with NCBI accession number NC_000005.10.

In some embodiments, the ferritin is soybean ferritin, preferably having an amino acid sequence with NCBI accession number NP_001236534.2.

In some embodiments, the ferritin is *Arabidopsis thaliana* ferritin, preferably having an amino acid sequence with NCBI accession number NP_195780.1.

In some embodiments, the ferritin is *Beta vulgaris* chloroplast ferritin, preferably having an amino acid sequence with NCBI accession number XP_010676813.1.

In some embodiments, the ferritin is *Rhizopus microsporus* ferritin, preferably having an amino acid sequence with NCBI accession number XP_023465511.1.

In some embodiments, the ferritin is *Encephalitozoon cuniculi* ferritin, preferably having an amino acid sequence with NCBI accession number XP_003887486.1.

In some embodiments, the ferritin is non-heme ferritin, preferably *Enterobacteriaceae* non-heme ferritin, and more preferably having an amino acid sequence with NCBI accession number WP_000917208.1.

In some embodiments, the ferritin is *Acinetobacter* ferritin, preferably having an amino acid sequence with NCBI accession number WP_002117082.1.

In some embodiments, the ferritin is *Campylobacter* ferritin, preferably having an amino acid sequence with NCBI accession number WP_002786063.1.

In some embodiments, the ferritin is *Bacteroides* ferritin, preferably having an amino acid sequence with NCBI accession number WP_010538613.1.

In some embodiments, the ferritin is *Pseudomonas aeruginosa* ferritin, preferably having an amino acid sequence as set forth in SEQ ID NO: 4.

In some embodiments, the ferritin is *Thermococcus barophilus* ferritin, preferably having an amino acid sequence with NCBI accession number NC_014804.1.

In some embodiments, the ferritin is *Pyrococcus yayanosii* ferritin, preferably having an amino acid sequence with NCBI accession number NC_015680.1.

In some embodiments, the ferritin is *Deinococcus-Thermus* ferritin, preferably having an amino acid sequence with NCBI accession number WP_038069286.1.

In some embodiments, the ferritin is *Actinobacteria* ferritin, preferably having an amino acid sequence with NCBI accession number NP_218358.1.

In some embodiments, the ferritin is *Proteobacteria* ferritin, preferably having an amino acid sequence with NCBI accession number NP_417795.1.

In some embodiments, the ferritin is *Escherichia coli* ferritin, preferably having an amino acid sequence as set forth in SEQ ID NO: 5.

In some embodiments, the ferritin is *Korarchaeota* ferritin, preferably having an amino acid sequence with NCBI accession number WP_012309233.1.

In some embodiments, the ferritin is *Pyrococcus furiosus* ferritin, preferably having an amino acid sequence with NCBI accession number WP_011011871.1.

In some embodiments, the ferritin is *Methanosarcina* ferritin, preferably having an amino acid sequence with NCBI accession number WP_048037772.1.

In some embodiments, the cross-linked ferritin further comprises hyaluronic acid and/or collagen.

Preferably, the volume ratio of the cross-linked ferritin to the hyaluronic acid is 1:5 to 5:1.

Preferably, the volume ratio of the cross-linked ferritin to the collagen is 1:5 to 5:1.

Preferably, the molar concentration ratio or mass ratio of the cross-linked ferritin to the hyaluronic acid is 1:5 to 5:1.

Preferably, the molar concentration ratio or mass ratio of the cross-linked ferritin to the collagen is 1:5 to 5:1.

The present disclosure further provides a method for preparing the cross-linked ferritin as described above, comprising the following steps:

preparing a cross-linked ferritin by a cross-linking reaction with ferritin of natural origin or a variant thereof, or the modified ferritin as described above, wherein the cross-linking reaction is a chemical cross-linking reaction or a biological cross-linking reaction.

In the present disclosure, when the cross-linking reaction is a chemical cross-linking reaction, the cross-linking reaction may comprise the following steps: mixing a neutral solution comprising ferritin of natural origin or a variant thereof, or a neutral solution comprising the modified ferritin as described above, with a cross-linking agent, and performing a cross-linking reaction to obtain the cross-linked ferritin.

Herein, after the cross-linking reaction, the process may further comprise mixing hyaluronic acid and/or collagen into the ferritin hydrogel.

Herein, after the cross-linking reaction, the process may further comprise neutralization, washing, and/or homogenization.

Herein, the cross-linking agent may be one or more selected from 1,4-butanediol diglycidyl ether (BDDE), genipin (GP), 1-ethyl-3-(3-dimethylaminopropyl)carbodiimide hydrochloride (EDC), N-hydroxysuccinimide (NHS), nordihydroguaiaretic acid (NDGA), and glutaraldehyde, preferably 1,4-butanediol diglycidyl ether (BDDE).

Herein, the volume percentage of the cross-linking agent relative to the neutral solution may be 3% to 30%, preferably 6%.

Herein, the cross-linking reaction may be performed in a constant temperature water bath, wherein the temperature of the cross-linking reaction may be 60 to 72°C, for example 60°C, and the duration of the cross-linking reaction may be 20 to 150 min.

Herein, the pH of the neutral cross-linked ferritin solution may be 8.

Herein, the concentration of the ferritin of natural origin or a variant thereof, or the modified ferritin as described above, may be 10 to 600 mg/mL, preferably 50 to 300 mg/mL, for example 100 mg/mL.

Herein, the neutralization may comprise acid washing to neutrality; the acid washing preferably comprises immersion washing with an acidic reagent; preferably, the acidic reagent is one or more selected from hydrochloric acid, sulfuric acid, and acetic acid, for example acetic acid.

Herein, the washing may comprise washing with a buffer salt solution; preferably, the buffer salt solution is one or more selected from phosphate buffer, physiological saline, Tris-HCl buffer, and HEPES, for example physiological saline; preferably, the concentration of the buffer salt solution is 10 to 100 mM, and the pH thereof is 6 to 8.

Herein, the duration of the washing may be 12 to 30 hours, for example 24 hours.

In the present disclosure, when the cross-linking reaction is a biological cross-linking reaction, the ferritin of natural origin may be cross-linked via disulfide bonds; preferably, a cross-linked ferritin is obtained by fusion expression of the ferritin of natural origin and a cysteine-containing polypeptide via genetic engineering.

Preferably, the natural origin comprises animal, plant, fungal, or bacterial origins, preferably animal origins, the animal being a vertebrate, preferably a mammal, and more preferably a human.

In a specific embodiment, the biological cross-linking reaction comprises, for example: mixing ferritin modified with a cysteine-containing polypeptide and β-mercaptoethanol, performing magnetic stirring, and after an initial reaction, centrifuging to remove the supernatant, washing the protein precipitate with PBS, then re-dissolving in a DMSO solution (pH 3 to 8), and performing magnetic stirring until the reaction is complete, to obtain the cross-linked ferritin.

The present disclosure further provides a use of the ferritin of natural origin or a variant thereof, or the modified ferritin as described above, in the preparation of a subcutaneous or tissue filler; wherein the variant has at least 80% identity, preferably at least 85%, 90%, 95%, 96%, 97%, 98%, or 99% identity, with the amino acid sequence of the ferritin of natural origin.

On the basis of conforming to common knowledge in the field, the above preferred conditions may be combined arbitrarily to obtain the preferred embodiments of the present disclosure.

The reagents and raw materials used in the present disclosure are all commercially available.

The positive and progressive effects of the present disclosure are as follows:

The present disclosure is the first to use cross-linked ferritin, obtained by cross-linking ferritin of natural origin or a variant thereof, or the modified ferritin, for subcutaneous and tissue filling. Compared with the raw materials of existing filling materials, the ferritin of the present disclosure, especially the ferritin obtained through genetic engineering, possesses good biocompatibility and non-immunogenicity. The cross-linked ferritin still maintains the unique intact multimeric structure (cage-shaped structure) of ferritin, and has characteristics such as good elasticity, high support, low swelling degree, resistance to degradation, resistance to migration, long residence time in vivo, and suitability for injectable use, which can achieve superior subcutaneous and tissue filling effects.

### BRIEF DESCRIPTION OF THE DRAWINGS

FIG. 1 and FIG. 2 are the microscopic morphological characterization results of the hydrogels obtained in Examples 1-2 and Comparative Examples 1-4.
FIG. 3 shows the microscopic morphological characterization results of the hydrogels obtained in Example 1 and Examples 10-15 after being diluted 100-fold.
FIG. 4 shows the characterization results of the enzymatic degradation resistance of uncross-linked LFn, HFn, BSA, and Col at 25°C.
FIG. 5 shows the characterization results of the enzymatic degradation rates at 37°C of the hydrogels obtained in Examples 1-2 (LFn, HFn), commercial HA, commercial Col, and the hydrogels obtained in Examples 10-13 (LFn-HA, HFn-HA, LFn-Col, and HFn-Col).
FIG. 6 shows the swelling levels of the hydrogels obtained in Examples 1-2 and Comparative Examples 3-4 after 24 hours of immersion washing.
FIG. 7 shows the swelling degrees of the hydrogels obtained in Examples 1-2 and Comparative Examples 3-4 after 24 hours of immersion washing.
FIG. 8 shows the elastic and viscous moduli of the hydrogels obtained in Examples 1-2 and Comparative Examples 3-4.
FIG. 9 and FIG. 10 show the gel point volumes and displacement levels of the hydrogels obtained in Examples 1-2 and Comparative Examples 3-4 after subcutaneous injection.
FIG. 11 shows the gel point maintenance results of the hydrogels obtained in Examples 1-2 and Comparative Examples 3-4 after subcutaneous injection.
FIG. 12 shows the inflammatory factor detection results of the hydrogels obtained in Examples 1-2 and Comparative Examples 3-4 after subcutaneous injection.

### DETAILED DESCRIPTION OF THE PREFERRED EMBODIMENT

The embodiments of the technical solution of the present disclosure will be described in detail below in conjunction with the accompanying drawings. The following examples are only intended to more clearly illustrate the technical solution of the present disclosure and are therefore provided merely as examples, and shall not be used to limit the scope of protection of the present disclosure. It should be noted that, unless otherwise stated, the technical or scientific terms used in the present application shall have the ordinary meanings understood by those skilled in the art to which the present disclosure belongs. Unless otherwise specified, the reagents used in the examples are all conventional commercially available products.

### Definitions

Ferritin is a common cage-shaped self-assembling protein widely existing in animals, plants, and microorganisms. Its primary function is to convert toxic divalent iron into trivalent iron and store it within an internal cavity, thereby regulating the iron metabolic balance of an organism.

The ferritin of the present disclosure refers to any ferritin capable of forming a ringshaped or cage-shaped structure, which may be ferritin of natural origin or modified ferritin, such as recombinantly expressed ferritin, or a variant thereof; it may be derived from prokaryotes, protists, fungi, plants, or animals, such as from bacteria, fungi, insects, reptiles, birds, amphibians, fish, and mammals; for example, from rodents, ruminants, non-human primates, or humans; such as mice, rats, guinea pigs, canines, cats, horses, sheep, monkeys, gorillas, and humans. From bacteria to humans, although the amino acid sequences of ferritin from different organisms differ greatly, their structures are similar, and all can form a ringshaped or cage-shaped protein shell structure.

In some examples or comparative examples, a neutral/basic cross-linking method was employed to prepare hydrogels: the cross-linking agent BDDE was taken at 6% by volume and slowly added to the protein solution; upon observation against light, the cross-linking agent was seen to slowly sink to the bottom or remain suspended in a filamentous form, layering with the protein solution. The pipette tip was suspended at the solution interface, followed by slow and gentle pipetting while observing against light until the layering and suspension phenomena completely disappeared, indicating thorough mixing. Cross-linking was then performed in a water bath at 60 to 72°C for 20 to 150 min.

In some examples or comparative examples, a biological cross-linking method was also employed to prepare hydrogels: the gene sequence of a ferritin subunit was fusion-expressed with a peptide chain via genetic engineering to obtain ferritin with the polypeptide linked to the outer surface; then, to this ferritin solution (1 mg/mL in 20 mM Tris-HCl), β-mercaptoethanol (β-ME) was added dropwise to a working concentration of 20 mmol/L, followed by magnetic stirring for 10 minutes. The reaction solution was collected and centrifuged for 10 minutes, the supernatant was removed, and the protein precipitate was washed twice with PBS. The collected protein precipitate was dissolved in a 6% DMSO solution (pH 3 to 8) at a protein solution concentration of 2 mg/mL, followed by magnetic stirring until the reaction was complete, thereby obtaining the cross-linked ferritin. The polypeptide herein may be any polypeptide chain containing cysteine.

The amino acid sequences of the ferritin used in the Examples or Comparative Examples of the present disclosure are specifically as follows:
1. Human light chain ferritin: 
SSQIRQNYSTDVEAAVNSLVNLYLQASYTYLSLGFYFDRDDVALEGVSHFFR
ELAEEKREGYERLLKMQNQRGGRALFQDIKKPAEDEWGKTPDAMKAAMALEKKLN
QALLDLHALGSARTDPHLCDFLETHFLDEEVKLIKKMGDHLTNLHRLGGPEAGLGEY
LFERLTLKHD [SEQ ID NO: 1];
2. Human heavy chain ferritin-1: 
MTTASTSQVRQNYHQDSEAAINRQINLELYASYVYLSMSYYFDRDDVALKN
FAKYFLHQSHEEREHAEKLMKLQNQRGGRIFLQDIKKPDCDDWESGLNAMECALHL
EKNVNQSLLELHKLATDKNDPHLCDFIETHYLNEQVKAIKELGDHVTNLRKMGAPE
SGLAEYLFDKHTLGDSDNES [SEQ ID NO: 2];
3. Mouse heavy chain ferritin-NCBI Reference Sequence: NP_034369.1;
4. Mouse light chain ferritin-GenBank: AAA37614.1;
5. *Drosophila melanogaster* heavy chain ferritin-NCBI Reference Sequence: NP_001247366.1;
6. *Drosophila melanogaster* light chain ferritin-NCBI Reference Sequence: NP_001263105.1;
*7. Caenorhabditis elegans* ferritin-NCBI Reference Sequence: NP_491198.1;
8. Oyster ferritin (PDB: 6LIJ): 
MAESQCRQNYHQESEAGINRQINMELYACYTYQSMAYYFDRDDVALPGFSK
FFKNSSDEEREHAEKLMKYQNKRGGRVVLQDIKKPDRDEWGTGLDAMQVALQLEK
TVNQSLLDLHKVADSHQDAQMCDFLETHYLEEQVNAIKEISDHITQLKRVGSGLGEY
EYDRRLDS* [SEQ ID NO: 3];
9. Human mitochondrial ferritin-NCBI Reference Sequence:NC_000005.10;
10. Soybean ferritin-NCBI Reference Sequence:NP_001236534.2;
11. *Arabidopsis thaliana* ferritin 1-NCBI Reference Sequence: NP_195780.1;
*12. Beta vulgaris* chloroplast ferritin-NCBI Reference Sequence: XP_010676813.1;
13. *Rhizopus microsporus* ferritin-NCBI Reference Sequence: XP_023465511.1;
14. *Encephalitozoon cuniculi* ferritin-NCBI Reference Sequence: XP_003887486.1;
15. *Enterobacteriaceae* non-heme ferritin-NCBI Reference Sequence: WP_000917208.1;
16. *Acinetobacter* bacterial ferritin-NCBI Reference Sequence: WP_002117082.1;
*17. Campylobacter* ferritin-NCBI Reference Sequence: WP_002786063.1;
18. *Bacteroides* ferritin-NCBI Reference Sequence: WP_010538613.1;
19. *Pseudomonas aeruginosa* ferritin (PDB: 6NLF): 
MKGDKKVIQHLNKILGNELIAINQYFLHSRMWNDWGLKRLGAHEYHESIDE
MKHADKLIERILFLEGLPNLQDLGKLLIGENTQEMLQCDLNLELKATKDLREAIVHCE
QVHDYVSRDLLKDILESEEEHIDYLETQLGLIQKVGLENYLQSHMHEDD* [SEQ ID
NO: 4];
20.*. Thermococcus barophilus* ferritin-NCBI Reference Sequence:NC_014804.1;
21. *Pyrococcus yayanosii* ferritin-NCBI Reference Sequence:NC_015680.1;
22. *Deinococcus-Thermus* ferritin-NCBI Reference Sequence: WP_038069286.1;
23. *Actinobacteria* ferritin-NCBI Reference Sequence:NP_218358.1;
24. *Proteobacteria* ferritin-NCBI Reference Sequence:NP_417795.1;
25. *Escherichia coli* ferritin: 
MLKPEMIEKLNEQMNLELYSSLLYQQMSAWCSYHTFEGAAAFLRRHAQEE
MTHMQRLFDYLTDTGNLPRINTVESPFAEYSSLDELFQETYKHEQLITQKINELAHAA
MTNQDYPTFNFLQWYVSEQHEEEKLFKSIIDKLSLAGKSGEGLYFIDKELSTLDTQN
[SEQ ID NO: 5];
26. *Korarchaeota* ferritin-NCBI Reference Sequence:WP_012309233.1;
27. *Pyrococcus furiosus* ferritin-NCBI Reference Sequence:WP_011011871.1;
28. *Methanosarcina* ferritin-NCBI Reference Sequence: WP_048037772.1;
29. Human heavy chain ferritin-2: 
MTTASTSQVRQNYHQDSEAAINRQINLELYASYVYLSMSYYFDRDDVALKN
FAKYFLHQSHEEREHAEKLMKLQNQRGGRIFLQDIKKPDCDDWESGLNAMECALHL
EKNVNQSLLELHKLATDKNDPHLCDFIETHYLIKELGDHVTNLRKMGAPESGLAEYL
FDKHTLGDSDNES [SEQ ID NO: 6];
30. Human heavy chain ferritin-3:

MTTASTSQVRQNYHQDSEAAINRQINLELYASYVYLSMSYYFDRDDVALKN
FAKYFLHQSHEEREHAEKLMKLQNQRGGRIFLQDIKKPDCDDWESGLNAMECALHL
EKNVNQSLLELHKLATDKNDPHLCDFIETHYLIKELGCHVTNLRKMGAPESGLAEYL
FDKHTLGDSDNES [SEQ ID NO: 7].

### Example 1

The ferritin used in this example is a modified human ferritin (human light chain ferritin, LFn), which contains 24 light chain subunits, each having an amino acid sequence as set forth in SEQ ID NO: 1.

The specific preparation steps for the human light chain ferritin (LFn) hydrogel are as follows:

1,4-butanediol diglycidyl ether (BDDE) was added at a volume percentage of 6% to a 200 mg/mL LFn aqueous solution with a pH of 8. After uniform mixing, the mixture was placed in a constant temperature water bath at a set reaction temperature of 60°C for a reaction time of 20 to 150 min to obtain a chemically cross-linked LFn protein gel. Then, the cross-linked LFn protein gel was neutralized to pH 7 by immersion washing with 0.05 M acetic acid, washed with physiological saline for 24 h, homogenized, and filled and sealed to obtain a chemically cross-linked LFn hydrogel (neutral cross-linking).

### Example 2

The ferritin used in this example is a modified human ferritin (human heavy chain ferritin, HFn), which contains 24 subunits, each having an amino acid sequence as set forth in SEQ ID NO: 2.

A chemically cross-linked HFn hydrogel was prepared under neutral conditions (neutral cross-linking) according to the same method as in Example 1.

### Example 3 - Example 9

The dispensed LFn protein solution was placed in the sample chamber of a freeze-dryer. After pre-freezing, sublimation drying, and analytical drying, LFn lyophilized protein was obtained. Different chemically cross-linked LFn protein gels were obtained by weighing LFn lyophilized protein according to the proportions shown in Table 1 and adding it to 1 mL each of a BDDE aqueous solution with a pH of 8, followed by uniform mixing and placement in a constant temperature water bath at a set reaction temperature of 60°C for a reaction time of 20 to 150 min.

Then, the cross-linked LFn protein gels were neutralized to pH 7 by immersion washing with 0.05 M acetic acid, washed with physiological saline for 24 h, homogenized, and filled and sealed.

**Table 1**

| No. | Weight of LFn lyophilized protein (mg) | Volume percentage of BDDE aqueous solution (%) |
|---|---|---|
| Example 3 | 300 | 3 |
| Example 4 | 300 | 6 |
| Example 5 | 300 | 9 |
| Example 6 | 400 | 6 |
| Example 7 | 550 | 3 |
| Example 8 | 550 | 6 |
| Example 9 | 550 | 9 |

### Example 10

The ferritin used in this example is a modified human ferritin (human light chain ferritin, LFn), which contains 24 subunits, each having an amino acid sequence as set forth in SEQ ID NO: 1.

50 mg of hyaluronic acid (HA, molecular weight: 200 KD) was added to a 200 mg/mL LFn solution with a pH of 8, and dissolved by gradual stirring at room temperature. BDDE was added at a volume percentage of 6% to the mixed solution of LFn and HA. After uniform stirring, the mixture was placed in a constant temperature water bath at 60°C for a reaction time of 1 to 1.5 hours to obtain a gel of LFn chemically cross-linked with HA, hereinafter referred to as LFn-HA. Then, the gel was neutralized to pH 7 by immersion washing with 0.5% HAC, washed with physiological saline for 15 hours, homogenized, and filled and sealed.

### Example 11

The ferritin used in this example is a modified human ferritin (human heavy chain ferritin, HFn), which contains 24 subunits, each having an amino acid sequence as set forth in SEQ ID NO: 2.

50 mg of hyaluronic acid (HA, molecular weight: 200 KD) was added to a 200 mg/mL HFn solution with a pH of 8, and dissolved by gradual stirring at room temperature. BDDE was added at a volume percentage of 6% to the mixed solution of HFn and HA. After uniform stirring, the mixture was placed in a constant temperature water bath at 60°C for a reaction time of 1 to 1.5 hours to obtain a gel of HFn chemically cross-linked with HA, hereinafter referred to as HFn-HA. Then, the gel was neutralized to pH 7 by immersion washing with 0.5% HAC, washed with physiological saline for 15 hours, homogenized, and filled and sealed.

### Example 12

The ferritin used in this example is a modified human ferritin (human light chain ferritin, LFn), which contains 24 subunits, each having an amino acid sequence as set forth in SEQ ID NO: 1.

20 mg of collagen (Col, pig-derived dry collagen product) was added to a 200 mg/mL LFn solution with a pH of 8, and dissolved by gradual stirring in a constant temperature water bath at 60°C. BDDE was added at a volume percentage of 6% to the mixed solution of LFn and Col. After uniform stirring, the mixture was placed in a constant temperature water bath at 60°C for a reaction time of 1.5 to 2 hours to obtain a gel of LFn chemically cross-linked with Col, hereinafter referred to as LFn-Col. Then, the gel was neutralized to pH 7 by immersion washing with 0.5% HAC, washed with physiological saline for 15 hours, homogenized, and filled and sealed.

### Example 13

The ferritin used in this example is a modified human ferritin (human heavy chain ferritin, HFn), which contains 24 subunits, each having an amino acid sequence as set forth in SEQ ID NO: 2.

20 mg of collagen (Col, pig-derived dry collagen product) was added to a 200 mg/mL HFn solution with a pH of 8, and dissolved by gradual stirring in a constant temperature water bath at 60°C. BDDE was added at a volume percentage of 6% to the mixed solution of HFn and Col. After uniform stirring, the mixture was placed in a constant temperature water bath at 60°C for a reaction time of 1.5 to 2 hours to obtain a gel of HFn chemically cross-linked with Col, hereinafter referred to as HFn-Col. Then, the gel was neutralized to pH 7 by immersion washing with 0.5% HAC, washed with physiological saline for 15 hours, homogenized, and filled and sealed.

### Example 14

The ferritin used in this example is a modified human ferritin (human heavy chain ferritin, HFn), which consists of 8 heavy chain subunits (hereinafter referred to as HFn-8mer), each having an amino acid sequence as set forth in SEQ ID NO: 6.

A chemically cross-linked HFn-8mer hydrogel was prepared under neutral conditions according to the same method as in Example 1.

### Example 15

The ferritin used in this example is a **modified** human ferritin (human heavy chain ferritin, HFn), which consists of 16 heavy chain subunits (hereinafter referred to as HFn-16mer), each having an amino acid sequence as set forth in SEQ ID NO: 7.

A chemically cross-linked HFn-16mer hydrogel was prepared under neutral conditions according to the same method as in Example 1.

### Comparative Example 1

A chemically cross-linked LFn hydrogel was prepared under basic conditions (basic cross-linking) at pH 12 (by dissolving 200 mg/mL LFn in NaOH to obtain a premix with a pH of 12) according to the method of Example 1.

### Comparative Example 2

A chemically cross-linked HFn hydrogel was prepared under basic conditions (basic cross-linking) at pH 12 (by dissolving 200 mg/mL HFn in NaOH to obtain a premix with a pH of 12) according to the method of Example 1.

### Comparative Example 3

100 mg of HA was dissolved in 1 mL of 75 mM NaOH and mixed uniformly to obtain a premix with a pH of 12. Then, BDDE was added to the premix at a volume percentage of 6%. After uniform mixing, the mixture was placed in a constant temperature water bath at a set reaction temperature of 45°C for a reaction time of 5 hours to obtain a chemically cross-linked HA gel. Then, the chemically cross-linked HA gel was neutralized to pH 7 by immersion washing with 0.05 M acetic acid, washed with physiological saline for 24 hours, homogenized, and filled and sealed to obtain a chemically cross-linked HA basic hydrogel.

### Comparative Example 4

100 mg of Col was dissolved in 1 mL of 0.25 M NaOH by heating at 60°C and mixed uniformly to obtain a premix with a pH of 12. Then, BDDE was added to the premix at a volume percentage of 6%. After uniform mixing, the mixture was placed in a constant temperature water bath at a set reaction temperature of 60°C for a reaction time of 5 to 12 hours to obtain a chemically cross-linked Col protein gel. Then, the chemically cross-linked Col protein gel was neutralized to pH 7 by immersion washing with 0.05 M acetic acid, washed with physiological saline for 24 hours, homogenized, and filled and sealed to obtain a chemically cross-linked Col basic hydrogel.

### Test and characterization results of Effect Example 1

The hydrogels obtained in the various examples and comparative examples were characterized as follows:
1. Microscopic morphology determination
   (1) For the hydrogels of Examples 1-2 and Comparative Examples 1-4, the microstructure was observed using a field emission scanning electron microscope.
   (2) For the hydrogels of Examples 1 and 10-15, after being diluted 100-fold, observation was performed using a field emission transmission electron microscope.
2. Enzymatic degradation resistance determination
   (1) For uncross-linked LFn, HFn, Col, and bovine serum albumin (BSA), enzymatic degradation resistance was tested by incubating with trypsin and collagenase respectively at 25°C for 2 hours and 24 hours, followed by SDS-PAGE detection.
   (2) For the hydrogels of Examples 1-2 (LFn, HFn), commercial HA filler (Biohyalux^{®} Black Gold), commercial Col filler (Helanni^{®}), and the hydrogels of Examples 10-13 (LFn-HA, HFn-HA, LFn-Col, and HFn-Col), the degradation rate was determined according to the following steps:
      A certain mass of the sample was weighed and recorded as m₀. The sample was placed in an EP tube, and 1 mL of different enzyme solutions (trypsin, collagenase) was added to each tube. After acting in a constant temperature shaker at 37°C and 100 r/min for different periods of time, the tubes were placed in a centrifuge at 8000 rpm for 5 minutes, and the supernatant was discarded by aspiration. The solution was dried, and the mass after enzymatic degradation, m_{d}, was recorded. The calculation formula for the sample degradation rate is: D=(m₀-m_{d})/m₀×100%.
3. Thermal stability determination
   For the hydrogels of Example 1, Example 2, and Examples 14-15, after incubating in pure water at 37°C, 60°C, 80°C, and 100°C for 30 minutes, respectively, the samples were placed in a centrifuge at 8000 rpm and centrifuged for 5 minutes to observe the precipitation of the samples.
4. Swelling degree determination
   For the hydrogels of Examples 1-2 and Comparative Examples 3-4, the weight of the dried material was weighed and recorded as m₀. The material was immersed in 2 mL of PBS at 37°C for 24 hours. After reaching equilibrium swelling, the PBS was removed. The gel was allowed to stand on a 45° inclined plane for 1 min and then weighed, recorded as m_{S}. The calculation formula for the swelling degree is S=(m_{S}-m₀)/m₀×100%.
5. Determination of elastic and viscous moduli
   For the hydrogels of Examples 1-2 and Comparative Examples 3-4, the viscoelasticity of the gels was determined using a Haake RS6000 (Thermo Fisher Scientific (China) Co., Ltd.) advanced rotational rheometer. Under the test temperature of 25°C, with a slit width of 1 mm and a vibration frequency in the range of 0.1 to 100 Hz, dynamic measurements of the elasticity and viscosity of the hydrogels were performed. The observation indexes were the storage modulus (G') and the loss modulus (G").
6. Observation of subcutaneous retention time and retention efficiency *in vivo*
   For the hydrogels of Examples 1-2 and Comparative Examples 3-4, 8-10 week old female nude mice were selected. Each hydrogel was subcutaneously injected into two points on the middle back of the mice, with 200 µL per point. The volume of the gel points was measured every 7 days within 30 days. After 30 days, the mice were sacrificed, and the remaining gel points were removed by dissection and weighed.
7. Observation of filling position displacement *in vivo*
   For the hydrogels of Examples 1-2 and Comparative Examples 3-4, 8-10 week old female nude mice were selected. Each hydrogel was subcutaneously injected into two points on the middle back of the mice, with 200 µL per point. The displacement distance of the gel points was measured every 7 days within 30 days.
8. Observation of inflammatory response *in vivo*
   For the hydrogels of Examples 1-2 and Comparative Examples 3-4, 8-10 week old Balb/c mice were selected. Each hydrogel was subcutaneously injected into two points on the back of the mice, with 200 µL per point. The mice were euthanized at 48 hours, and inflammatory factors in local tissues were detected by multiplex assay, using mice without subcutaneous injection (Normal) as a blank control.
9. Injection experience score based on clinicians

For the hydrogels of Example 1 and Examples 3-9, injectability evaluations were performed by 5 clinical doctors from the Department of Dermatological Cosmetology, using Juvéderm^{®} VOLUMA^{®} as a control.

### The results are as follows:

In the following results, all hydrogels were prepared according to the aforementioned methods, except that the actual concentration used during subcutaneous injection was 100 mg/mL.

### 1. Appearance and microscopic morphological characterization results

The cross-linked samples containing HFn appear brown. Due to its ferroxidase activity, it may participate in *in vivo* iron metabolism when used as a filler material.

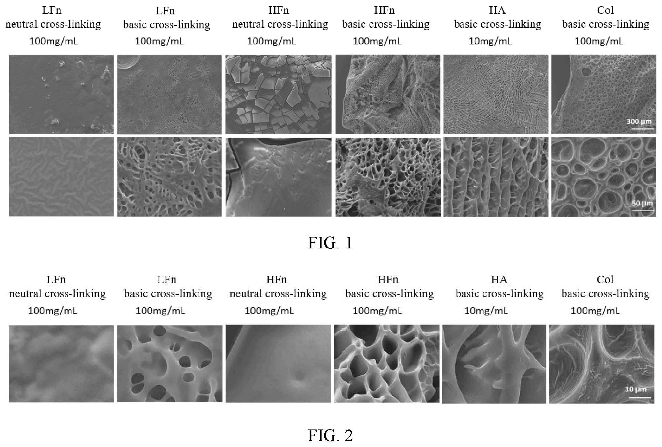

The microscopic morphologies of the hydrogels of Examples 1-2 and Comparative Examples 1-4 are shown in FIG. 1 and FIG. 2. The results indicate that after cross-linking under neutral conditions, LFn or HFn both exhibit a dense state; whereas cross-linking under basic conditions results in cross-linked products of monomers after depolymerization, exhibiting a porous structure.

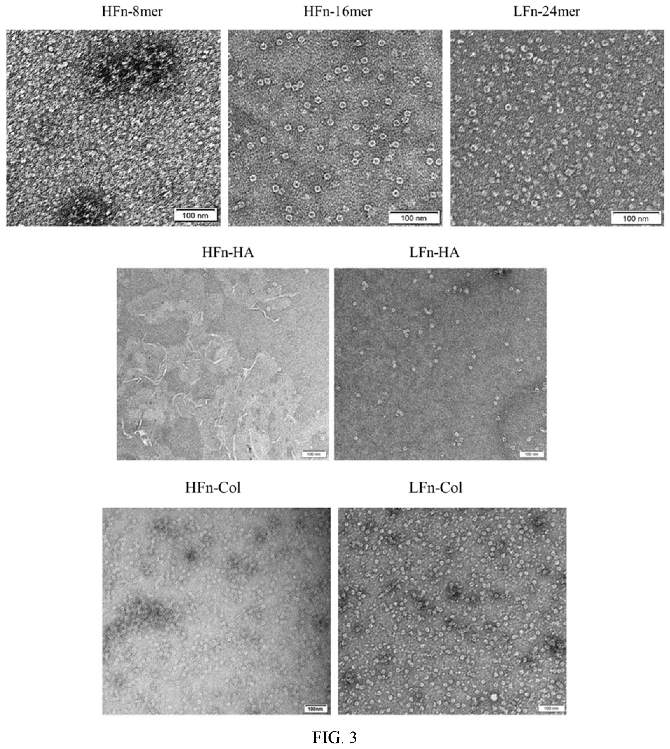

The microscopic morphologies of the hydrogels of Example 1 and Examples 10-15 after being diluted 100-fold are shown in FIG. 3. The results indicate that the wild-type LFn-24mer (i.e., Example 1) has a complete cage-shaped structure; HFn-8mer (Example 14) has a semi-bowl structure and does not form a cage-shaped structure; and HFn-16mer (Example 15) has a cage-shaped structure formed by joining two semi-bowls. Furthermore, LFn-HA, LFn-Col, HFn-HA, and HFn-Col in Examples 10-13 all exhibit cage-shaped structures, indicating that the further cross-linking of LFn or HFn with HA or Col does not affect the original cage-shaped structure.

### 2. Enzymatic degradation resistance characterization results

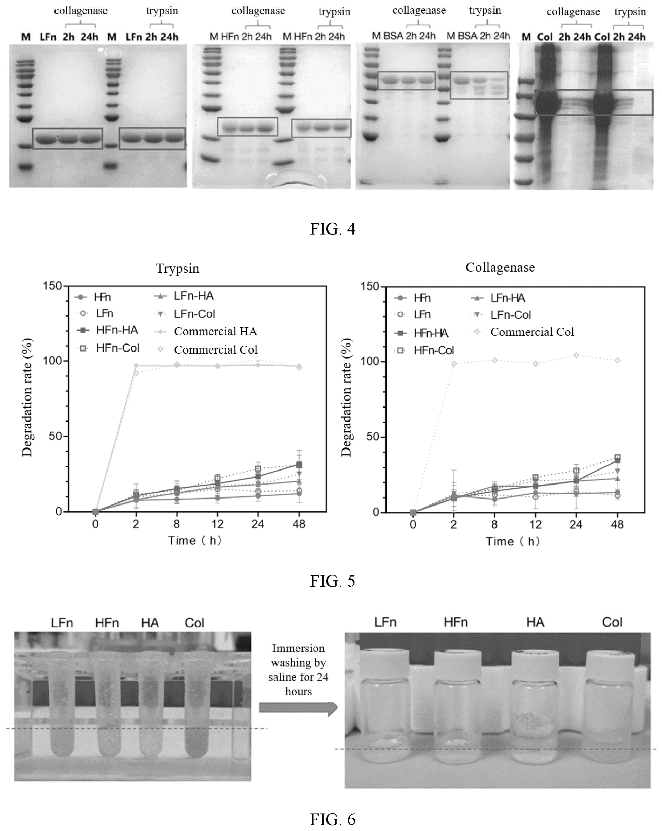

The enzymatic degradation resistance of uncross-linked LFn, HFn, Col, and bovine serum albumin (BSA) is shown in the SDS-PAGE results in FIG. 4. The results show that collagenase can only specifically decompose collagen and has no significant degradation effect on uncross-linked LFn, HFn, and BSA; under trypsin catalysis for 2 to 24 hours, the degradation of uncross-linked LFn and HFn is not significant. This indicates that uncross-linked LFn and HFn possess resistance to collagenase and trypsin degradation.

The enzymatic degradation rates at 37°C for the hydrogels of Examples 1-2 (LFn, HFn), commercial HA filler (Biohyalux^{®} Black Gold), commercial Col filler (Helanni^{®}), and the hydrogels of Examples 10-13 (LFn-HA, HFn-HA, LFn-Col, and HFn-Col) are shown in FIG. 5. The results show that the degradation rates of LFn and HFn under the action of trypsin and collagenase are significantly lower; whereas commercial HA and commercial Col can be almost completely degraded by trypsin and collagenase within 2 hours at 37°C.

### 3. Thermal stability determination results

Upon observation, after testing the hydrogels of Example 1, Example 2, and Examples 14-15 at 80°C, the hydrogels of HFn-8mer (Example 14) and HFn-16mer (Example 15) showed significantly more protein precipitation. This indicates that the hydrogels of HFn-8mer and HFn-16mer have relatively poor thermal stability due to structural issues.

### 4. Swelling level and swelling degree determination results

The swelling level results of the hydrogels of Examples 1-2 and Comparative Examples 3-4 are shown in FIG. 6. The results indicate that all samples underwent varying degrees of swelling after immersion washing. The higher the cross-linking degree, the lower the water absorption. From the visual appearance, HA underwent a high degree of swelling, Col swelled slightly, and LFn and HFn did not undergo significant swelling, indicating that the cross-linking degrees of LFn and HFn are relatively high. The swelling levels follow the order: LFn, HFn < Col < HA. Excessive swelling of a hydrogel under physiological conditions will lead to a decrease in its mechanical strength and may simultaneously affect the growth of surrounding tissues or cause abnormal physiological indicators in the body.

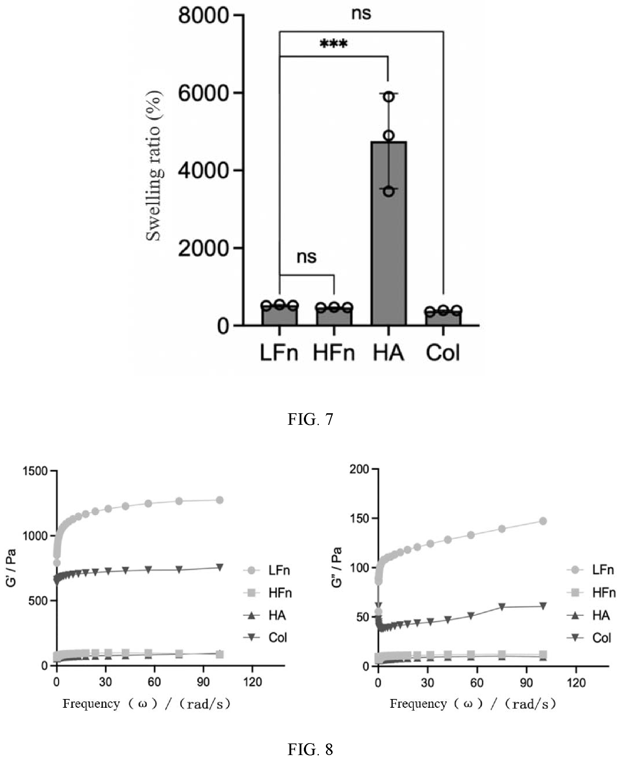

The results of the swelling degrees of the hydrogels of Examples 1-2 and Comparative Examples 3-4 are shown in FIG. 7. The results show that the swelling degrees of LFn (527.1%) and HFn (469.7%) have no significant difference from that of Col (608.7%), while they are significantly lower than that of HA (4756.2%), suggesting that the mechanical strength of LFn and HFn is close to that of Col and higher than that of HA.

### 5. Determination results of elastic and viscous moduli

The detection results of the elastic modulus and viscous modulus of the hydrogels of Examples 1-2 and Comparative Examples 3-4 are shown in FIG. 8. G' represents the elastic modulus and G" represents the viscous modulus; G' > G" indicates that the sample is in a liquid state. After the samples stabilized, the elastic modulus of LFn was higher than 1000 Pa and that of Col was higher than 600 Pa, while both HFn and HA were lower than 200 Pa; the viscous modulus of LFn was higher than 100 Pa and that of Col was higher than 30 Pa, while both HFn and HA were lower than 20 Pa. Both the elasticity and viscosity follow the order of LFn > Col > Hfn or HA, indicating that LFn has the optimal elasticity and viscosity, and HFn is close to HA.

### 6. Observation results of subcutaneous retention time and filling position displacement in vivo

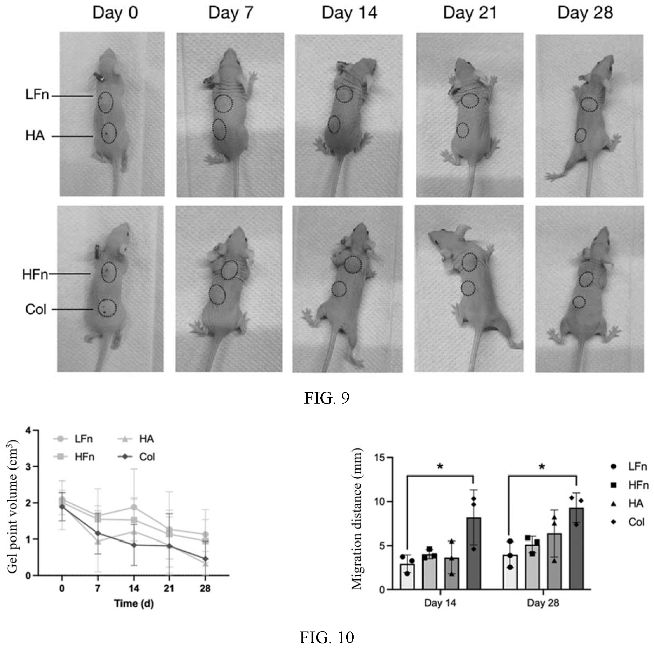

For the hydrogels of Examples 1-2 and Comparative Examples 3-4, the *in vivo* subcutaneous degradation levels (retention capacity/extended area) and displacement levels are shown in FIG. 9. The hydrogels were subcutaneously injected into two points on the middle back of mice, with 200 µL per point. The length, width, height, and displacement were measured (as shown in FIG. 10). The extended area was calculated as 4/3π(abc). The results indicate that the retention capacity of ferritin in the middle back is higher than that of HA and Col. After 28 days, the remaining gel point volumes for LFn (1.12 cm³) and HFn (0.95 cm³) were similar, while HA and Col were degraded and reduced to 0.34 cm³ and 0.47 cm³, respectively. The relative motility in the middle back is weak. Within 28 days, the subcutaneous displacement of ferritin was at a similar level to that of HA (6.38 mm), and significantly lower than the 9.30 mm observed for Col. Among them, the degree of displacement for LFn was relatively the smallest (3.98 mm) (as shown in FIG. 10).

### 7. Observation results of in vivo retention efficiency

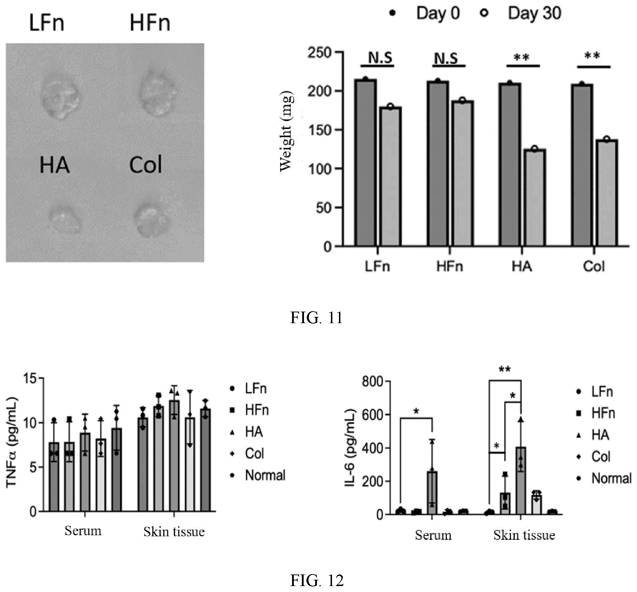

For the hydrogels of Examples 1-2 and Comparative Examples 3-4, the results of the subcutaneous hydrogel retention experiment in mice are shown in FIG. 11, where the image on the left shows the results after 30 days. The results indicate that within one month, the LFn and HFn gel points remained stably retained without significant degradation. After 30 days, Col and HA showed significant degradation, with HA exhibiting the most pronounced degradation effect. The retention status of LFn and HFn was superior to that of HA and Col.

### 8. Observation results of in vivo inflammatory response

For the hydrogels of Examples 1-2 and Comparative Examples 3-4, the detection of inflammatory factors for the subcutaneous hydrogels in mice is shown in FIG. 12. At 48 hours after subcutaneous injection in Balb/c mice, serum was collected to measure inflammatory factors, and gel point skin tissue was homogenized to measure inflammatory factors. Compared with the Normal group, subcutaneous filling with LFn or HFn did not cause up-regulation of the inflammatory factors IL-6 and TNF-α in the serum; subcutaneous filling with LFn did not cause up-regulation of the inflammatory factors IL-6 and TNF-α in the skin tissue, suggesting that LFn does not induce a systemic inflammatory response. After HA injection, IL-6 in the serum and skin tissue was up-regulated by 11.1-fold and 21.2-fold, respectively.

### 9. Results of injection experience scores based on clinicians

The results of the injection experience scores for the hydrogels of Example 1 and Examples 3-9, and Juvéderm^{®} VOLUMA^{®} are shown in Tables 2 and 3. The meanings of the scores are as follows:
"+" represents: Stable injection is possible under a certain force; the needle occasionally has slight blockage or resistance; the material possesses a certain degree of viscoelasticity.
"++" represents: Injection is relatively smooth; there is basically no blockage or resistance; the material has acceptable viscoelasticity.
"+++" represents: Easy to inject without resistance; the needle is not easily blocked; the material has good viscoelasticity.

**Table 2**

| | 200 mg/mL LFn: BDDE (6%) cross-linked | 300 mg/mL LFn: BDDE (6%) cross-linked | 400 mg/mL LFn: BDDE (6%) cross-linked | 550 mg/mL LFn: BDDE (6%) cross-linked | Juvéderm^{®} VOLUMA^{®} |
|---|---|---|---|---|---|
| Clinician A | ++ | +++ | +++ | +++ | ++ |
| Clinician B | + | ++ | +++ | +++ | ++ |
| Clinician C | ++ | ++ | ++ | +++ | ++ |
| Clinician D | + | ++ | +++ | +++ | +++ |
| Clinician E | ++ | ++ | +++ | +++ | ++ |

**Table 3**

| | 300 mg/mL | 300 mg/mL | 300 mg/mL | 550 mg/mL | 550 mg/mL | 550 mg/mL | Juvéderm^{®} VOLUMA^{®} |
|---|---|---|---|---|---|---|---|
| | LFn: BDDE (3%) cross-linked | LFn: BDDE (6%) cross-linked | LFn: BDDE (9%) cross-linked | LFn: BDDE (3%) cross-linked | LFn: BDDE (6%) cross-linked | LFn: BDDE (9%) cross-linked | |
| Clinician A | ++ | +++ | +++ | +++ | +++ | +++ | ++ |
| Clinician B | ++ | ++ | +++ | ++ | +++ | +++ | ++ |
| Clinician C | +++ | ++ | +++ | ++ | +++ | +++ | ++ |
| Clinician D | ++ | ++ | +++ | ++ | +++ | +++ | +++ |
| Clinician E | +++ | ++ | +++ | ++ | +++ | +++ | ++ |

The results indicate that in the LFn hydrogels, the viscosity of the hydrogel improves as the protein concentration increases; additionally, in the LFn hydrogels, the viscosity of the hydrogel improves as the volume ratio of BDDE increases.

## Claims

1. A modified ferritin comprising a plurality of light chain subunits or heavy chain subunits of ferritin of natural origin, wherein the plurality of light chain subunits or the plurality of heavy chain subunits form a multimer, preferably a cage-shaped multimer.

2. The modified ferritin according to claim 1, wherein the amino acid sequence of the light chain subunit is as set forth in SEQ ID NO: 1 or has at least 80% identity with the sequence as set forth in SEQ ID NO: 1, and the amino acid sequence of the heavy chain subunit is as set forth in SEQ ID NO: 2, 6, or 7 or has at least 80% identity with the sequence as set forth in SEQ ID NO: 2, 6, or 7;
preferably, the modified ferritin comprises 8, 16, 24, or 48 light chain subunits or 8, 16, 24, or 48 heavy chain subunits; and/or, the natural origin comprises animal, plant, fungal, or bacterial origins, preferably animal origins, the animal being a vertebrate, preferably a mammal, more preferably a human;
more preferably, the modified ferritin consists of 24 light chain subunits forming the cage-shaped multimer, or consists of 24 heavy chain subunits forming the cage-shaped multimer.

3. A cross-linked ferritin, which is a cross-linked ferritin formed by cross-linking ferritin of natural origin or a variant thereof, wherein the cross-linked ferritin retains the multimeric structure of the ferritin of natural origin or the variant thereof; or is a cross-linked ferritin formed by cross-linking the modified ferritin according to claim 1 or 2, wherein the cross-linked ferritin retains the multimeric structure of the modified ferritin; wherein the variant has at least 80% identity with the amino acid sequence of the ferritin of natural origin;
preferably, the cross-linked ferritin is in the form of a hydrogel or microsphere structure.

4. The cross-linked ferritin according to claim 3, wherein the swelling degree of the cross-linked ferritin is lower than 1000%, preferably lower than 600%, and more preferably lower than 500%;
and/or, the elastic modulus of the cross-linked ferritin is higher than 100 Pa, preferably higher than 600 Pa, and more preferably higher than 1000 Pa;
and/or, the viscous modulus of the cross-linked ferritin is higher than 10 Pa, preferably higher than 30 Pa, more preferably higher than 50 Pa, and even more preferably higher than 100 Pa;
and/or, the natural origin comprises animal, plant, fungal, or bacterial origins, preferably animal origins, the animal being a vertebrate, preferably a mammal, and more preferably a human;
preferably, the cross-linking is performed via a biological cross-linking reaction or a chemical cross-linking reaction.

5. The cross-linked ferritin according to claim 3, wherein the cross-linked ferritin further comprises hyaluronic acid and/or collagen;
preferably, the volume ratio of the cross-linked ferritin to the hyaluronic acid is 1:5 to 5:1; or the volume ratio of the cross-linked ferritin to the collagen is 1:5 to 5:1;
or, the molar concentration ratio or mass ratio of the cross-linked ferritin to the hyaluronic acid is 1:5 to 5:1; or, the molar concentration ratio or mass ratio of the cross-linked ferritin to the collagen is 1:5 to 5:1.

6. A method for preparing the cross-linked ferritin according to any one of claims 3 to 5, wherein the method comprises the following steps:
preparing a cross-linked ferritin by a cross-linking reaction with ferritin of natural origin or a variant thereof, or the modified ferritin according to claim 1 or 2, wherein the cross-linking reaction is a chemical cross-linking reaction or a biological cross-linking reaction.

7. The method according to claim 6, wherein when the cross-linking reaction is a chemical cross-linking reaction, the cross-linking reaction comprises the following steps:
mixing a neutral solution comprising ferritin of natural origin or a variant thereof, or a neutral solution comprising the modified ferritin according to claim 1 or 2, with a cross-linking agent, and performing a cross-linking reaction to obtain the cross-linked ferritin;
and/or, after the cross-linking reaction, the method further comprises mixing hyaluronic acid and/or collagen into the cross-linked ferritin;
and/or, after the cross-linking reaction, the method further comprises neutralization, washing, and/or homogenization;
when the cross-linking reaction is a biological cross-linking reaction, the ferritin of natural origin is cross-linked via disulfide bonds; preferably, the cross-linked ferritin is obtained by fusion expression of the ferritin of natural origin and a cysteine-containing polypeptide via genetic engineering;
preferably, the natural origin comprises animal, plant, fungal, or bacterial origins, preferably animal origins, the animal being a vertebrate, preferably a mammal, and more preferably a human.

8. The method according to claim 7, wherein when the cross-linking reaction is a chemical cross-linking reaction, the method comprises one or more of the following conditions:
(1) the cross-linking agent is one or more selected from 1,4-butanediol diglycidyl ether, genipin, 1-ethyl-3-(3-dimethylaminopropyl)carbodiimide hydrochloride, N-hydroxysuccinimide, nordihydroguaiaretic acid, and glutaraldehyde, preferably 1,4-butanediol diglycidyl ether;
(2) the volume percentage of the cross-linking agent relative to the neutral solution is 3% to 30%, preferably 6%;
(3) the cross-linking reaction is performed in a constant temperature water bath; wherein the temperature of the cross-linking reaction is preferably 60 to 72°C, more preferably 60°C; and the duration of the cross-linking reaction is preferably 20 to 150 minutes;
(4) the pH of the neutral solution is 8;
(5) the concentration of the ferritin of natural origin or a variant thereof, or the modified ferritin according to claim 1 or 2, is 10 to 600 mg/mL, preferably 50 to 300 mg/mL, for example 100 mg/mL.

9. The method according to claim 7 or 8, wherein the method comprises one or more of the following conditions:
(1) the neutralization comprises acid washing to neutrality; the acid washing preferably comprises immersion washing with an acidic reagent; preferably, the acidic reagent is one or more selected from hydrochloric acid, sulfuric acid, and acetic acid, more preferably acetic acid;
(2) the washing comprises washing with a buffer salt solution; preferably, the buffer salt solution is one or more selected from phosphate buffer, physiological saline, Tris-HCl buffer, and HEPES; and/or the concentration of the buffer salt solution is 10 to 100 mM, and the pH thereof is 6 to 8;
(3) the duration of the washing is 12 to 30 hours.

10. Use of ferritin of natural origin or a variant thereof, or the modified ferritin according to claim 1 or 2, in the preparation of a subcutaneous or tissue filler, wherein the variant has at least 80% identity with the amino acid sequence of the ferritin of natural origin;
preferably, the natural origin comprises animal, plant, fungal, or bacterial origins, preferably animal origins, the animal being a vertebrate, preferably a mammal, and more preferably a human.
